Europäisches Patentamt

European Patent Office

Office européen des brevets

(19)

(11) **EP 0 613 686 B1**

(12) **FASCICULE DE BREVET EUROPEEN**

(45) Date de publication et mention
de la délivrance du brevet:
**16.08.2001 Bulletin 2001/33**

(51) Int Cl.⁷: **A61K 31/495**, A61P 13/12

(21) Numéro de dépôt: **94400404.3**

(22) Date de dépôt: **25.02.1994**

(54) **Utilisation de la trimétazidine pour l'obtention de médicaments destinés au traitement des troubles liés à la néphrotoxicité de la cyclosporine A.**

Verwendung von Trimetazidin zur Herstellung von Medikamenten für die Behandlung von Gesundheitsstörungen, die durch die Nephrotoxizität von Cyclosporin A bedingt sind.

Use of trimetazidine for the preparation of medicaments for the treatment of troubles due to the nephrotoxicity of cyclosporin A

(84) Etats contractants désignés:
**AT BE CH DE DK ES FR GB GR IE IT LI LU NL PT SE**

(30) Priorité: **26.02.1993 FR 9302171**

(43) Date de publication de la demande:
**07.09.1994 Bulletin 1994/36**

(73) Titulaire: **ADIR ET COMPAGNIE**
**92415 Courbevoie Cédex (FR)**

(72) Inventeurs:
- **Crevat, Aimé**
  **F-13001 Marseille (FR)**
- **Chauvet-Monges, Anne-Marie**
  **F-13001 Marseille (FR)**
- **Albengres, Edith**
  **F-75013 Paris (FR)**
- **Tillement, Jean-Paul**
  **F-77590 Bois le Roi (FR)**
- **Le Ridant, Alain**
  **F-92200 Neuilly sur Seine (FR)**
- **Lepagnol, Jean**
  **F-28210 Chaudon (FR)**

(56) Documents cités:
- **CARDIOVASC. DRUG REVIEWS vol. 6, no. 4 , 1989 pages 292 - 312 C. HARPEY ET AL. 'Trimetazidine, a cellular anti-ischemic agent'**
- **'DICTIONNAIRE VIDAL' 1987 , OVP , PARIS * page 1685 * * Vastarel 20mg ***
- **J UROL (UNITED STATES), APR 1993, VOL. 149, NO. 4, PAGE(S) 915-7, CREAGH T ET AL 'Pharmacological manipulation of acute cyclosporin ischemic renal injury with trimetazidine.'**
- **82ND ANNUAL SCIENTIFIC SESSION OF THE JAPANESE SOCIETY OF INTERNAL MEDICINE, NAGOYA, JAPAN, APR. 4-6, 1985.;& JPN J MED,, VOL. 24 (4). 1985 (RECD. 1986). 335. CODEN: (NO ISSN): JJMDA KUMADA M ET AL 'THE EFFECTS OF ANTI-PLATELET AGENTS ON RENAL FUNCTIONS AND THE MODES OF THEIR ANTI-PLATELET ACTION IN PRIMARY GLOMERULONEPHRITIS'**
- **REN FAIL (UNITED STATES), 1992, VOL. 14, NO. 4, PAGE(S) 485-91, ALIVANIS P ET AL 'Reduction of CyA nephrotoxicity by nifedipine during and after experimental in situ renal preservation.'**
- **NIPPON JINZO GAKKAI SHI (JAPAN), FEB 1985, VOL. 27, NO. 2, PAGE(S) 209-19, SUZUKI Y ET AL 'Antinephritic effect of trimetazidine on nephrotic-type and crescentic-type anti-GBM nephritis in rats]'**
- **CHEMICAL ABSTRACTS, vol. 99, no. 23, 5 Décembre 1983, Columbus, Ohio, US; abstract no. 187406, K. TANASE ET AL. 'Effects of trimetazidine on renal function in acute renal venous congestion with dogs' page 66 ;colonne 1 ; & YAKURI TO CHIRYO vol. 11, no. 9 , 1983 pages 3611 - 3619**

- **SEMIN THORAC CARDIOVASC SURG (UNITED STATES), APR 1990, VOL. 2, NO. 2, PAGE(S) 198-203, NEILD GH 'Cyclosporin nephrotoxicity.'**
- **ADV EXP MED BIOL (UNITED STATES), 1990, VOL. 264, PAGE(S) 383-8, CATROUX P ET AL 'Antilipoperoxydant effect of trimetazidine in post ischaemic acute renal failure in the rat.'**
- **AM HEART J (UNITED STATES), FEB 1993, VOL. 125, NO. 2 PT 2, PAGE(S) 566-71, VAN ZWIETEN PA 'Protective effects of calcium antagonists in different organs and tissues.'**
- **J UROL (UNITED STATES), NOV 1991, VOL. 146, NO. 5, PAGE(S) 1441-5, CREAGH TA ET AL 'A canine model of cyclosporine enhanced ischemic renal injury.'**

**Description**

La présente invention a pour objet l'utilisation de la trimétazidine et de ses sels physiologiquement tolérables pour l'obtention de médicaments destinés au traitement des troubles liés à l'utilisation thérapeutique des immunosuppresseurs.

Il a été décrit, dans la littérature, une classe d'immunosuppresseurs dont le chef de file est la ciclosporine. Cette classe comporte d'autres analogues de la ciclosporine et des composés structurellement différents mais agissant de manière similaire, cf. S.L. Schreiber and G.R. Crabtree, The mechanism of action of cyclosporin A and FK 506, Immunology today, vol 13, n° 4, p 136-142 (1992).

Cette classe d'immunosuppresseurs est porteuse d'une toxicité cellulaire s'observant notamment au niveau de la respiration des cellules de différents organes (reins, foie, coeur...) et au niveau de la fonctionnalité de certains de ces organes, principalement de la fonctionnalité rénale.

Il a été trouvé, dans les services de la demanderesse que la trimétazidine protège ces organes des altérations induites par la ciclosporine, restaure leur fonction respiratoire cellulaire physiologique et la fonctionnalité des organes atteints, principalement le rein.

Dans ce but, il a été mesuré, dans un premier temps, la consommation d'oxygène sur des fractionnements cellulaires, et dans un deuxième temps, il a été démontré la capacité protectrice de la Trimétazidine vis à vis des effets néphrotoxiques de la ciclosporine.

Jusqu'à présent, pour limiter cette toxicité, on utilisait au niveau clinique des hypotenseurs de la classe des antagonistes calciques, lesquels sont difficiles d'utilisation du fait des problèmes cardiovasculaires inhérents : hypotension, oedème, etc... phénomènes observés notamment avec la Nifédipine (DCI) cf. Goodman and Gilman's, The pharmacological Basis of Therapeutics, Seventh Edition, Mac Millan, p 819-820.

Ces inconvénients n'existent pas avec la trimétazidine et ses sels physiologiquement tolérables qui n'ont pas d'effet hypotenseur chez l'homme.

Par ailleurs, la trimétazidine n'altère pas l'effet immunologique de la ciclosporine et ne corrige donc que son effet respiratoire cellulaire indésirable.

Jusqu'alors, la trimétazidine était connue comme agent anti-ischémique utilisé comme tel en clinique, cf. C. Harpey et al., Cardiovascular Drug Reviews, vol 6. n° 4. pp 292-312 (1989) Raven Press Ltd New York.

La présente invention a également pour objet les compositions pharmaceutiques contenant comme principe actif la trimétazidine ou un de ses sels physiologiquement tolérables, mélangé ou associé à un ou plusieurs excipients pharmaceutiques appropriés.

Les compositions pharmaceutiques obtenues sont présentées avantageusement sous des formes pharmaceutiques convenant notamment pour l'administration par voie orale telle que, par exemple, les comprimés, dragées, gélules ou solutions buvables, et par voie parentérale sous forme de solutés injectables.

Elles se présentent généralement sous forme dosée contenant de 10 à 100 mg de principe actif.

La posologie varie selon l'âge et le poids du patient, la voie d'administration, la nature de l'indication thérapeutique et des traitements associés, et s'échelonne de 10 à 100 mg de principe actif par prise, de 1 à 10 fois par jour selon la dose unitaire utilisée.

La présente invention est illustrée par l'étude pharmacologique ci-après.

**ETUDE PHARMACOLOGIQUE**

Tous les tests qui suivent ont été réalisés en utilisant comme principe actif le dichlorhydrate de trimétazidine c'est à dire le dichlorhydrate de 1-[(2,3,4-triméthoxyphényl)méthyl] pipérazine, et comme agent immunosuppresseur la ciclosporine A.

**A/ Protection de la fonction respiratoire cellulaire altérée par la ciclosporine A.**

Elle est évaluée par la mesure des vitesses de respiration :

La consommation d'oxygène est mesurée polarographiquement à l'aide d'une microélectrode de Clark reliée à un oxygraphe Gilson.
Cette consommation est mesurée sur la fraction $P_2$ (crude Pellet) isolée à partir de foies de rat selon la technique de G. Johnson et H. Lardy, Methods of Enzymology, Estabook RW and Pullman Eds, New York Academic Press (1967), 10, 94-96.

**Méthodologie :**

La cellule de mesure (1,8 ml) est thermostatée à 25 °C. La fraction $P_2$ (1,5 mg de protéine) est suspendue dans un milieu de respiration contenant : saccharose 0,25 M, $KH_2PO_4$ 4mM,, Roténone 1μM. Le substrat utilisé est le succinate de sodium (concentration finale : 6 mM). La phosphorylation oxydative correspondant à l'état 3 est déclenchée par l'addition d'ADP (concentration finale 0,1 mM).

La consommation d'oxygène durant l'état 3 permet de calculer le rapport P/O qui indique le nombre de molécules d'ADP phosphorylées par atome d'oxygène consommé. Le contrôle respiratoire (CR) est le rapport entre les vitesses de consommation d'oxygène à l'état 3 ($V_3$) et à l'état 4 ($V_4$). (L'état 4 correspondant à la vitesse de respiration après que tout l'ADP ait été phosphorylé). Les vitesses sont exprimées en nanomoles d'oxygène consommées par mg de protéine de la fraction $P_2$ et par minutes.

L'action de la ciclosporine A (1μM) sur $V_3$ et P/O est évaluée après incubation de la fraction $P_2$ pendant 10 minutes à 4°C avec la ciclosporine A. Les mêmes expériences sont réalisées en présence de diverses doses de dichlorhydrate de trimétazidine qui sont ajoutées dans la cuve de mesure immédiatement avant le début de la mesure. Les valeurs témoins de $V_3$ et P/O sont obtenues selon le même protocole expérimental sans ajout de ciclosporine A ni de trimétazidine.

Chaque série de mesures destinée à tester la relation dose-effet de la trimétazidine est réalisée avec des fractions $P_2$ provenant de la même extraction et dans une même série, chaque mesure est répétée deux fois. Les résultats donnés dans les tableaux ci-après représentent la moyenne de trois séries de mesures.

L'effet correcteur de la trimétazidine (TMZ) sur l'action de la ciclosporine A (Cis A) au niveau de $V_3$ et P/O est exprimé en pourcentage de restauration calculé comme suit :

$$\% \text{ de restauration} = \frac{(V_3 \text{ ou P/O})(\text{Cis A} + \text{TMZ}) - (V_3 \text{ ou P/O}) \text{ Cis A}}{(V_3 \text{ ou P/O})\text{valeur témoin} - (V_3 \text{ ou P/O}) \text{ Cis A}}$$

Tous ces résultats sont obtenus en présence de 50 nmoles de $Ca^{++}$ dans la cuve de mesure.

**Résultats :**

Ils sont regroupés dans les tableaux 1 et 2 ci-après.
Le tableau 1 montre l'action de la ciclosporine A (Cis A) sur $V_3$ et P/O.
Le tableau 2 rapporte les effets de restauration du dichlorhydrate de trimétazidine (TMZ, 2HCl) sur $V_3$ et P/O altérés par la ciclosporine A.

Tableau 1

| Cis A Concentration en μM | % de dimunition de | |
|---|---|---|
| | $V_3$ | P/O |
| 0,5 | 4 ± 1 | 10 ± 2 |
| 1,0 | 14 ± 3 | 19 ± 3 |
| 1,5 | 17 ± 3 | 28 ± 3 |

Ce tableau montre l'effet de la ciclosporine A sur $V_3$ et P/O (valeur moyenne ± SEM). Le temps d'incubation est de 10 minutes. Les pourcentages sont calculés par rapport aux valeurs témoins (sans ciclosporine A) sans $Ca^{++}$.

Tableau 2

| TMZ, 2HCL concentration en μM | % de restauration de | |
|---|---|---|
| | $V_3$ | P/O |
| 0,025 | 10 ± 5 | 13 ± 3 |
| 0,05 | 26 ± 7 | 18 ± 5 |
| 0,1 | 39 ± 7 | 41 ± 9 |
| 0,2 | 49 ± 8 | 54 ± 7 |

Tableau 2   (suite)

| TMZ, 2HCL concentration en $\mu$M | % de restauration de | |
|---|---|---|
| | $V_3$ | P/O |
| 0,4 | 64 $\pm$ 6 | 83 $\pm$ 9 |
| 0,8 | 80 $\pm$ 5 | 92 $\pm$ 9 |

Ce tableau montre le pourcentage de restauration de $V_3$ et P/O par le dichlorhydrate de trimétazidine après action de la ciclosporine A à la concentration de 1 $\mu$M en présence de 50 nmoles de $Ca^{++}$ (valeurs moyennes $\pm$ SEM).

**Conclusion :**

Des résultats précédents, on peut déduire que la trimétazidine, sous forme de dichlorhydrate, restaure la fonction respiratoire cellulaire préalablement altérée par la ciclosporine A.

**B/ Inhibition par la trimétazidine des effets néphrotoxiques de la ciclosporine A**

La ciclosporine A et les composés appartenant à sa classe pharmacologique sont utilisés en thérapeutique afin d'éviter les phénomènes de rejet après transplantations d'organes (cf : N. Engl. J. Med 309, 809-815, 1983) mais également en dermatologie, dans le traitement du psoriasis notamment.
Cependant, son utilisation thérapeutique présente l'inconvénient d'une toxicité rénale.
Nous avons démontré que la trimétazidine permet d'inhiber ce type d'effet néphrotoxique induit par la ciclosporine.

**Matériel et méthodes :**

Deux groupes de huit rats mâles Sprague-Dawley ont été soumis soit à l'action de la trimétazidine, soit de son véhicule (groupe témoin).
La dose administrée a été de 10 mg/kg/jour de dichlorhydrate de trimétazidine pendant sept jours, par voie sous-cutanée.
Les reins des rats des deux groupes ont ensuite été isolés et perfusés par 1,2 mg/minute de ciclosporine A pendant cinq minutes.
Les paramètres révélant les effets secondaires de la ciclosporine A (débit urinaire, débit de filtration glomérulaire et flux plasmatique rénal) ont ensuite été mesurés pendant quarante minutes.
Les protocoles expérimentaux utilisés sont ceux déjà décrits dans la littérature (cf : Lab. Invest. 58, 163-171, 1988 ; Clin. Sci. Mol. Med 55, 513-521, 1978 et J. Biol. Chem. 158, 581-591, 1945) ; les résultats obtenus ont été analysés statistiquement.

**Résultats :**

Pour les animaux témoins, il est observé après perfusion de la ciclosporine A une chute considérable et statistiquement significative des valeurs des paramètres mesurés (40 à 60 %). Par contre, pour les animaux prétraités par le dichlorhydrate de trimétazidine, la chute est minime et non significative ; ce qui démontre l'effet protecteur de la trimétazidine vis à vis de tels effets néphrotoxiques induits par la ciclosporine A . Les résultats sont rassemblés dans le tableau ci-après :

|  |  | P1 | P2 | P3 |
|---|---|---|---|---|
| **GROUPE TÉMOIN** | Avant ciclosporine A | $73 \pm 8$ | $0,780 \pm 0,106$ | $30 \pm 2$ |
|  | Après ciclosporine A (Λ) | $44 \pm 13*$ | $0,350 \pm 0,092**$ | $12 \pm 4**$ |
| **GROUPE TRAITÉ AU DICHLORHYDRATE DE TRIMÉTAZIDINE** | Avant ciclosporine A | $68 \pm 12$ | $0,735 \pm 0,110$ | $27 \pm 4$ |
|  | Après ciclosporine A (Λ) | $63 \pm 10$ | $0,678 \pm 0,086$ | $25 \pm 2$ |

(Λ) : 40 minutes après la perfusion de ciclosporine A
P1 : Débit urinaire (µl/mn)
P2 : Débit de filtration glomérulaire (ml/mn/g de rein)
P3 : Flux plasmatique rénal (ml/mn)
* : $p \leq 0,05$ vs témoins avant ciclosporine A
** : $p \leq 0,01$ vs témoins avant ciclosporine A

**Revendications**

1. Utilisation pour l'obtention de médicaments destinés au traitement des troubles liés à la néphrotoxicité de la cyclosporine A, de la trimétazidine et de ses sels physiologiquement tolérables.

2. Utilisation, pour l'obtention de médicaments, selon la revendication 1, du dichlorhydrate de trimétazidine.

**Claims**

1. Use of trimetazidine and physiologically tolerable salts thereof for obtaining medicaments intended for the treatment of disorders related to the nephrotoxicity of cyclosporin A.

2. Use of trimetazidine dihydrochloride for obtaining medicaments according to claim 1.

**Patentansprüche**

1. Verwendung von Trimetazidin und seinen physiologisch verträglichen Salzen für die Herstellung von Arzneimitteln zur Behandlung von Störungen, die mit der Nierengiftwirkung von Cyclosporin A verbunden sind.

2. Verwendung nach Anspruch 1 von Trimetazidin-Dihydrochlorid.